# EUROPEAN PATENT SPECIFICATION

(11) **EP 3 066 467 B1**
(45) Date of publication and mention of the grant of the patent: **16.03.2022**
(21) Application number: 14792815.4
(22) Date of filing: 03.11.2014
(51) Int. Cl.: G01N 33/50, G01N 33/574

(54) **USE OF A REAGENT FOR THE LYSIS OF ERYTHROCYTES AS WELL AS METHODS AND KITS RELATING THERETO**
VERWENDUNG EINES REAGENZ ZUR LYSE VON ERYTHROZYTEN SOWIE VERFAHREN UND KITS, DIE DIESE BETREFFEN
UTILISATION D'UN RÉACTIF DE LYSE D'ÉRYTHROCYTES AINSI QUE LES PROCÉDÉS ET KITS ASSOCIÉS

(30) Priority: 05.11.2013 EP 13005219
(43) Date of publication of application: 14.09.2016
(73) Proprietor: Roche Diagnostics GmbH, 68305 Mannheim (DE); F. Hoffmann-La Roche AG, 4070 Basel (CH)
(72) Inventor: ANKENBAUER, Waltraud, 68167 Mannheim (DE); FROEHLICH, Thomas, 82377 Penzberg (DE); FROEHNER, Stefanie, 82377 Penzberg (DE)
(74) Representative: Teschemacher, Andrea
(86) International application number: PCT/EP2014/073519
(87) International publication number: WO 2015/067546

(56) References cited:
- EP-A2- 0 795 751
- US-A1- 2002 115 118
- US-A1- 2006 172 300
- INOUE M ET AL: "An in vivo model of priming of antigen-specific human CTL by Mo-DC in NOD/Shi-scid IL2rgamma<null> (NOG) mice", IMMUNOLOGY LETTERS, ELSEVIER BV, NL, vol. 126, no. 1-2, 22 September 2009 (2009-09-22), pages 67-72, XP027597376, ISSN: 0165-2478, DOI: 10.1016/J.IMLET.2009.08.001 [retrieved on 2009-08-12]
- CHERNYSHEV ET AL: "Erythrocyte lysis in isotonic solution of ammonium chloride: Theoretical modeling and experimental verification", JOURNAL OF THEORETICAL BIOLOGY, ACADEMIC PRESS, LONDON, GB, vol. 251, no. 1, 22 October 2007 (2007-10-22), pages 93-107, XP022486917, ISSN: 0022-5193, DOI: 10.1016/J.JTBI.2007.10.016

## Description

The present disclosure relates to use of a reagent for the lysis of erythrocytes and a method of lysing erythrocytes.

Methods of diagnosis and analysis usually aim at being minimal invasive, technically robust and universally applicable. Blood analysis has been used as a diagnostic and analytic tool for many years, as blood is obtainable without difficulty and the analysis of its components is usually relatively easy and automatable.

Blood is made up of several different kinds of cells and other compounds, including various salts and certain proteins. In vertebrates, blood is essentially composed of blood cells suspended in blood plasma. Plasma, which constitutes 55% of blood fluid, contains dissipated proteins, glucose, mineral ions, hormones, carbon dioxide (plasma being the main medium for excretory product transportation), and blood cells themselves. The blood cells are mainly red blood cells (also called RBCs or erythrocytes), white blood cells (also called WBCs or leukocytes) and platelets (thrombocytes). The most abundant cells in vertebrate blood are red blood cells. Humans have about 4 to 6 million erythrocytes per microliter of blood, whereas there are about 4,000-11,000 white blood cells and about 150,000-400,000 platelets in each microliter of human blood. Erythrocytes are mainly responsible for the transport of respiratory gases. Leukocytes are cells of the immune system and found throughout the body, including the blood and lymphatic system. Thrombocytes circulate in the blood of mammals and are involved in hemostasis, leading to the formation of blood clots.

In order to analyze components, particularly cellular components, of blood other than erythrocytes, it is desirable to remove erythrocytes, which is not easy due to their high number. For this, hemolysis, i.e. lysis or rupture of erythrocytes, has been used.

Many methods and protocols for erythrocyte lysis have been developed, some of which are detailed in the following:
Ammonium chloride was described as a penetrating salt enabling lysis of erythrocytes in whole blood. The classical ammonium chloride lysis buffer contains 150 mM NH₄Cl, 1 mM KHCO₃ and 0.1 mM EDTA. Erythrocytes can be depleted quantitatively using this buffer, but around 30 % or more leukocytes also become lost. (see e.g. Meryman H. Red Cell Structure and Function 1969; 352-367, Sass M. Am J Physiol. 1979;236(5):C238-43, Claus R. et al. Folia Hematol. 1985; 5: 683-688, Terstappen et al. J Immun Methods. 1989: 103-112).

US 7,678,583 B2 discloses a method to lyse erythrocytes quantitatively using a buffer with the core components piperidine or pyrrolidine hydrochlorid, potassium hydrogen carbonate and carbonic anhydrase. This lysis buffer composition is described to result in a higher leukocyte discovery rate with a quantitative erythrocyte depletion compared to the lysis procedure with ammonium chloride.

US 5,840,515 describes a method for isolating and differenting leukocytes in a blood sample by lysis of erythrocytes with a solution whose osmolality and pH have been adjusted to maintain leukocyte integrity and containing saponin and inhibition of the lysis by diluting the sample with a solution having a substantially similar composition but not containing saponin. The reagent is composed of 0.1 to 2 g/l of saponin and having an osmolality between 200 and 400 milliosmoles and a pH between 6 and 8.

DE 102008032501 A1 relates to a general lysis reagent which can be used for nucleic acid analysis and which contains a non-ionic tenside and a polymer acting as thickening agent. To analyze nucleic acids in leukocytes, erythrocytes are depleted using the following lysis buffer composition: 320 mM Saccharose, 50 mM Tris/Cl pH 7.5, 5 mM MgCl₂, 1% Triton X-100.

US 5,155,044 discloses a lytic reagent system for selective chemical treatment of whole blood comprising an acidic aqueous solution consisting essentially of a diluent, a lytic reagent selected from the group consisting of formic acid, acetic acid and their respective mixtures; the relative concentration of the lytic reagent in said acidic aqueous solution being sufficient to effect partitioning of a whole blood sample into a lysed red cell fraction and an essentially intact leukocyte fraction in such a state as to allow differential analysis of at least five subpopulations of such leukocytes; and a clarification effective amount of saponin in the range of from about 0.05 to about 0.2 percent.

EP 0 795 751 A2 teaches that leucocytes can be quickly and selectively separated from erythrocytes by subjecting a whole blood sample to a series of steps including lysing the erythrocytes and washing the remaining leucocytes with a solution containing ammonium chloride and a carboxylic acid or a metal carboxylate.

US 2006/172300 A1 a method for identifying the presence of BBB-specific protein/fragment in endothelial cells of brain capillaries, comprising inter alia treating enzymaticcaly digested endothelial cells of brain capillaries with a lysis buffer that essentially destroys present erythrocytes and apoptotic cells and maintains at least 70% of the endothelial cells of brain capillaries in vital form. The hypotonic buffer used should have an ionic strength of 0.1-0.2 M, contain monovalent and bivalent anions and cations, respectively, and buffer in a pH range of 7.0-8.0.

All of these described erythrocytes lysis protocols and also protocol variations beyond of the cited ones above (hypotonic lysis, detergent-dependent lysis, ammonium-based lysis, acetic acid-based lysis) have been tested and have the disadvantage that at least 20-30 % of the leukocytes are lost during the erythrocyte lysis procedure (see Example 7). Therefore, these procedures are not appropriate for a quantitative erythrocyte depletion procedure combined with a high leukocyte recovery rate, e.g. of at least 90 %.

Accordingly, it was an object of the present disclosure to provide means and methods for quantitative erythrocyte depletion combined with a high viable leukocyte recovery rate. Preferably, the means and methods should not influence leukocyte morphology. Additionally, reactions having a negative influence on cell viability or on experiments following erythrocyte lysis (e.g. PCR or FACS or enzymatic reactions) should be avoided. Surprisingly, the object was solved by a new reagent to be used in the lysis of erythrocytes, the reagent being an aqueous solution comprising or consisting of HEPES ((4-(2-hydroxyethyl)-1-piperazineethanesulfonic acid), NH₄⁺/NH₃, a chelating agent selected from the group consisting of EGTA (ethylene glycol tetraacetic acid), BAPTA (1,2-bis(o-aminophenoxy)ethane-N,N,N',N'-tetraacetic acid) DTPA (diethylene triamine pentaacetic acid), EDTA (ethylenediamine tetraacetate) and NTA (N,N-bis(carboxymethyl)glycine) and optionally CO₃²⁻/CO₃⁻, wherein the final concentration during lysis of erythrocytes is in the range of
- from 2.5 mmol/l to 12 mmol/l HEPES,
- from 60 mmol/l to 120 mmol/l NH₄⁺/NH₃,
- from 0.04 mmol/l to 0.8 mmol/l chelating agent, and
- from 0.15 mmol/l to 0.8 mmol/l CO₃²⁻/CO₃⁻, if present.

The newly developed lysis buffer is based on ammonium chloride as main lysis component. But in comparison to the established ammonium chloride buffers, concentration of ammonium chloride is lower in the developed buffer and other buffer components, such as HEPES, a chelating agent and KHCO₃, are added to the reagent mixture. This buffer composition enables quantitative erythrocyte depletion with a high leukocyte recovery rate, preferably of at least 90 % (see Example 4) in contrast to established methods, in which the leukocyte recovery rate was considerably lower (see Examples 1 and 2). Additionally, components suspected of having adverse effects on cell viability or experiments following erythrocyte lysis are not present.

In comparison to the generally used ammonium chloride lysis protocols, the developed means and methods allow to maintain a physiologically stable pH value between 6.8 and 7.4 during the lysis procedure (see Example 6). When using the common ammonium chloride lysis buffer procedures a pH value around 8.0 is measured. Under such conditions also non-erythrocyte blood cells die due to the non-physiological pH value.

However, leukocyte isolation from whole blood enabled by an erythrocyte depletion procedure is an important step to study multiple physiological and pathophysiological blood cell and blood linked phenomena, e.g. immunological evaluations, such as determination of inflammatory and immune state; oncological approaches, such as investigation of different types of leukemia or detection of circulating tumor cells in blood; cardiovascular approaches, such as investigation of circulating endothelial cells in blood; non clinical safety approaches; or flow cytometry approaches, such as those based on every physiological and pathophysiological applications to study nucleated cells in blood (leukocytes and others).

Accordingly, in a first aspect, the present disclosure relates to the use of a reagent for the lysis of erythrocytes, the reagent being an aqueous solution comprising or consisting of HEPES ((4-(2-hydroxyethyl)-1-piperazineethanesulfonic acid), NH₄⁺/NH₃ and a chelating agent optionally CO₃²⁻/CO₃⁻, wherein the final concentration during lysis of erythrocytes is in the range of
- from 2.5 mmol/l to 12 mmol/l HEPES,
- from 60 mmol/l to 120 mmol/l NH₄⁺/NH₃, and
- from 0.04 mmol/l to 0.8 mmol/l chelating agent,
wherein the chelating agent is selected from the group consisting of EGTA (ethylene glycol tetraacetic acid), BAPTA (1,2-bis(o-aminophenoxy)ethane-N,N,N',N'-tetraacetic acid), DTPA (diethylene triamine pentaacetic acid), EDTA (ethylenediamine tetraacetate) and NTA (N,N-bis(carboxymethyl)glycine).

The reagent may further comprise from 0.15 mmol/l to 0.8 mmol/l CO₃²⁻/CO₃⁻.

As detailed above, the reagent may be used for the lysis of erythrocytes. In accordance with the present disclosure, the reagent is an aqueous solution, i.e. a solution based on water. The reagent comprises or consists of the components listed above in aqueous solution, which means that the aqueous solution consists of these components only (consisting of) or encompasses also at least one further component (comprising).

In a specific embodiment as presented herein, the reagent does not comprise one or more or all of the following agents: piperidine or salt thereof, pyrrolidine or salt thereof, carbonic anhydrase, saponin, a tenside, and a polymer acting as thickening agent.

During lysis, the final concentration of the components listed above is as defined above. For use in lysis, a ready-made reagent may be used which is mixed with a source comprising erythrocytes. In this case, the dilution of the reagent has to be considered when preparing the ready-made reagent. If e.g. blood or a blood product is used a source, the reagent may be added to thereto (or vice versa), thus diluting the reagent. Typical dilutions may be from 1:10 to 10:1 (source : reagent), thus requiring before dilution a 1.1-fold to 11-fold stock reagent, respectively. X-fold stock reagent means that the concentrations in the components of the reagent before dilution are increased by factor X relative to the final concentrations during lysis as indicated herein.

Red blood cell's lysis is usually achieved by a mechanism found on the osmotic balance disturbance. Erythrocytes normally are shaped as biconcave disks. In hypotonic environment, spherisation of the cells and subsequent increase in volume can be observed. When membrane tension exceeds a critical value, membrane ruptures, thus lysing erythrocytes. Accordingly, lysis in the present context refers to the breaking down of a cell by osmotic mechanisms that compromise its integrity.

One component of the reagent is HEPES ((4-(2-hydroxyethyl)-1-piperazineethanesulfonic acid), which is a zwitterionic organic chemical buffering agent. HEPES is widely used in biology, biochemistry, such as in cell culture, largely because it is better at maintaining physiological pH despite changes in carbon dioxide concentration when compared to other buffers. The dissociation of water decreases with falling temperature, but the dissociation constants (pK) of many other buffers do not change much with temperature. HEPES is like water in that its dissociation decreases as the temperature decreases. This makes HEPES a more effective buffering agent for maintaining enzyme structure and function at low temperatures. A buffer is most effective when the pH is equal to the pKa of that buffer, and most efficient when in the range of one pH unit above and below that value. HEPES is commonly used to maintain pH levels in cell media. In comparison to the inorganic sodium bicarbonate buffer system, HEPES is more suitable for buffering in the physiological pH range of 7.2-7.6. HEPES is a "Good" buffer, containing both positive and negative ionizable groups, where the secondary and tertiary amine groups provide the positive charge and the negative charges are offered by the sulfonic and carboxylic acid groups. Usually, HEPES is added to media at concentrations of 15 mM to 25 mM, however, in the present disclosure the concentration of HEPES is lower.

Another component of the reagent is an ammonia (NH₃) / ammonium (NH₄⁺) buffer. The final concentration of ammonia + ammonium is in the above range of from 60 mmol/l to 120 mmol/l. Usually, the buffer is prepared of ammonium chloride and ammonia, wherein the ratio of both components varies depending on the intended pH. The pKa of the buffer system is 9.25.

Additionally, the reagent comprises a chelating agent. A chelating agent is an agent complexing ions, which reduces their concentrations. Usually, the ions are metal ions, such as Ca, Mg, Fe, Zn and Cu, but non-metal ions, such as P, may be also complexed. By forming stable water soluble complexes with multivalent (metal) ions, chelating agents prevent undesired interaction by blocking normal reactivity of (metal) ions. In the present invention the chelating agent is selected from the group consisting of EGTA (ethylene glycol tetraacetic acid), BAPTA (1,2-bis(o-aminophenoxy)ethane-N,N,N',N'-tetraacetic acid) DTPA (diethylene triamine pentaacetic acid), EDTA (ethylenediamine tetraacetate) and NTA (N,N-bis(carboxymethyl)glycine), wherein the reagent comprising EDTA as a chelating agent represents a particularly advantageous embodiment. EDTA is an example of a very common chelating agent which has nitrogen atoms and short chain carboxylic groups and is used as an anticoagulant.

Optionally, another component of the reagent is a carbonate (CO₃²⁻) / hydrogen carbonate (HCO₃⁻) buffer. The final concentration of carbonate + hydrogen carbonate is in the above range of from 0.15 mmol/l to 0.8 mmol/, if present. Usually, the buffer is prepared by dissolving salts of carbonate and hydrogen carbonate, such as potassium or sodium salts, in water, wherein the ratio of carbonate / hydrogen carbonate varies depending on the pH. The pKa of bicarbonate is 6.1, yielding the best buffering capacity at a pH of 5.1-7.1.

In a further specific embodiment, one or more components as defined above are present in a final concentration during lysis in the range of
- from 3 mmol/l to 11 mmol/l HEPES, preferably from 3 mmol/l to 10 mmol/l HEPES, more preferably from 3.5 mmol/l to 4.5 mmol/l HEPES,
- from 70 mmol/l to 100 mmol/l NH₄⁺/NH₃, preferably from 75 mmol/l to 85 mmol/l NH₄⁺/NH₃,
- from 0.05 mmol/l to 0.5 mmol/l chelating agent, preferably from 0.06 mmol/l to 0.2 mmol/l chelating agent, more preferably from 0.07 mmol/l to 0.1 mmol/l chelating agent, and/or
- from 0.3 mmol/l to 0.6 mmol/l CO₃²⁻/CO₃⁻, preferably from 0.3 mmol/l to 0.5 mmol/l CO₃²⁻/CO₃⁻, more preferably from 0.35 mmol/l to 0.45 mmol/l CO₃²⁻/CO₃⁻, if present.

In a particular embodiment, the components as defined above are present in a final concentration during lysis in the range of from 3 mmol/l to 10 mmol/l HEPES, of from 70 mmol/l to 100 mmol/l NH₄⁺/NH₃, of from 0.05 mmol/l to 0.5 mmol/l chelating agent and of from 0.3 mmol/l to 0.6 mmol/l CO₃²⁻/CO₃⁻.

In yet another particular embodiment, the components as defined above advantageously are present in a final concentration during lysis in the range of from 3 mmol/l to 10 mmol/l HEPES, of from 75 mmol/l to 85 mmol/l NH₄⁺/NH₃, of from 0.06 mmol/l to 0.2 mmol/l chelating agent and of from 0.3 mmol/l to 0.5 mmol/l CO₃²⁻/CO₃⁻, particularly wherein the chelating agent is ethylene diamine tetraacetic acid (EDTA).

In yet another particular embodiment and with particular advantage, the components as defined above are present in a final concentration during lysis in the range of from 3.5 mmol/l to 4.5 mmol/l HEPES, of from 75 mmol/l to 85 mmol/l NH₄⁺/NH₃, of from 0.07 mmol/l to 0.1 mmol/l chelating agent and of from 0.35 mmol/l to 0.45 mmol/l CO₃²⁻/CO₃⁻, particularly wherein the chelating agent is ethylene diamine tetraacetic acid (EDTA).

In yet a further specific embodiment, the pH of the reagent is in the range of from 6.4 to 7.7, in a particular embodiment advantageously the pH of the reagent is in the range of from 6.7 to 7.4, in yet another particular embodiment and with particular advantage the pH of the reagent is in the range of from 6.8 to 7.3. In chemistry, pH is a measure of the activity of the (solvated) hydrogen ion. Pure water has a pH very close to 7 at 25°C. Solutions with a pH less than 7 are said to be acidic and solutions with a pH greater than 7 are basic or alkaline. The pH of blood is usually slightly basic with a value in the range of from pH 7.35 to pH 7.45. In a further specific embodiment the pH is maintained in the range of from 6.4 to 7.7, particularly 6.7 to 7.4, more specifically 6.8 to 7.3 during lysis of erythrocytes, which is advantageous in order to maintain viability of non-erythrocyte cells. Accordingly, the components as defined above are present in a final concentration during lysis in the range of from 3 mmol/l to 10 mmol/l HEPES, of from 70 mmol/l to 100 mmol/l NH₄⁺/NH₃, of from 0.05 mmol/l to 0.5 mmol/l chelating agent and of from 0.3 mmol/l to 0.6 mmol/l CO₃²⁻/CO₃⁻ and the pH of the reagent is in the range of from 6.4 to 7.7, preferably 6.7 to 7.4, more preferably 6.8 to 7.3.

Still more specifically, the components as defined above are advantageously present in a final concentration during lysis in the range of from 3 mmol/l to 10 mmol/l HEPES, of from 75 mmol/l to 85 mmol/l NH₄⁺/NH₃, of from 0.06 mmol/l to 0.2 mmol/l chelating agent and of from 0.3 mmol/l to 0.5 mmol/l CO₃²⁻/CO₃⁻ and the pH of the reagent is in the range of from 6.4 to 7.7, particularly 6.7 to 7.4, more specifically 6.8 to 7.3, particularly wherein the chelating agent is ethylene diamine tetraacetic acid (EDTA).

In a highly specific embodiment and with particular advantage, the components as defined above are present in a final concentration during lysis in the range of from 3.5 mmol/l to 4.5 mmol/l HEPES, of from 75 mmol/l to 85 mmol/l NH₄⁺/NH₃, of from 0.07 mmol/l to 0.1 mmol/l chelating agent and of from 0.35 mmol/l to 0.45 mmol/l CO₃²⁻/CO₃⁻ and the pH of the reagent is in the range of from 6.4 to 7.7, particularly 6.7 to 7.4, more specifically 6.8 to 7.3, particularly wherein the chelating agent is ethylene diamine tetraacetic acid (EDTA).

In yet further a specific embodiment, the reagent is used in the isolation of cells other than erythrocytes (also referred to as non-erythrocyte cells) from a sample comprising erythrocytes, particularly from a blood sample or blood product.

For providing a blood sample, blood needs to be taken from a subject. Particularly for mammals, this may be conveniently performed by taking venous blood from the subject. Venous blood may be obtained by venipuncture from the mammal, wherein usually only a small sample, e.g. 3 ml to 10 ml sample, of blood is adequate for the use in the present disclosure. Blood is most commonly obtained from the median cubital vein, on the anterior forearm (the side within the fold of the elbow). This vein lies close to the surface of the skin, and there is not a large nerve supply. Most blood collection in the industrialized countries is done with an evacuated tube system consisting of a plastic hub, a hypodermic needle, and a vacuum tube. However, blood may also be obtained by any other method known to the skilled person.

After isolation of the blood, blood may be processed, e.g. by adding an anti-coagulant. After having been obtained and optionally further processed, the blood may be immediately used for analysis or stored as known to the person skilled in the art. The feature "blood product" in the present disclosure refers to a product derived from blood, wherein blood has been processed to obtain the blood product. Examples include blood with additives (such as heparin), packed red blood cells, erythrocyte concentrates, thrombocyte concentrates, granulocyte concentrates, blood stem cell preparations, etc.

In a particular embodiment, blood is isolated as follows: Blood is taken from a subject and collected in container intended for blood collection. Those containers are commercially available and may be used in the method as presented herein. Usually, they comprise an anti-coagulant such as EDTA. An exemplary container is a routine EDTA Vacutainer tubes (BD Biosciences, Heidelberg, Germany). In order stabilize cell membranes of WBCs, suitable agents known to the skilled person may be added. Furthermore, a buffer solution adapted for stabilisation at neutral conditions may be present. Blood samples may be gently inverted. Thereafter, the sample may be immediately used or stored until used.

Alternatives sources for mixtures of cells including red blood cells include biopsy samples, bone marrow, urine, stool, or body fluids with red blood cells as a "contaminant".

In a specific embodiment, the erythrocytes or the sample containing erythrocytes are obtained from a mammal, particularly from a mammalian domestic animal, such as cat, dog, rabbit, or guinea pig, or farm animal, such as cow, horse, goat, sheep, swine or camel. In a very specific embodiment, the erythrocytes or the sample containing erythrocytes are obtained from a human.

As detailed above, the reagent may be used in the detection, concentration or isolation of cells other than erythrocytes from a sample comprising erythrocytes. The sample may be any suitable sample, but a blood sample or a sample derived from blood, e.g. a processed blood sample or a blood product, is a specific embodiment which can be used with particular advantage. The reagent may be used to detect, concentrate or isolate cells other than erythrocytes from a mixture of cells comprising erythrocytes. The reagent is used for the lysis of erythrocytes, thus increasing the percentage of other cells in the sample. Accordingly, cells other than erythrocytes are concentrated by the lysis. Further steps of detection, concentration or isolation of cells of interest may be combined with the lysis, including centrifugation such as differential centrifugation or gradient centrifugation, labeling of cells of interest and subsequent detection or separation of the same, cell sorting, e.g. by FACS and so on. Suitable methods for detection, concentration or isolation of cells of interest are well known to the skilled person.

Evidently, the type of the cells of interest depends from the source chosen (blood etc., see above) and the intended application or technical field. In general, the cell of interest might be any cell present in the source or sample chosen. In a specific embodiment, the cells other than erythrocytes are leukocytes, B cells, T cells, eosinophils, circulating endothelial cells, or cancer cells such as circulating tumor cells, particularly circulating tumor cells or circulating tumor microemboli, which are of particular relevance for diagnostic purposes.

The present disclosure is particularly helpful for the detection or isolation of rare cells, particularly wherein in the population the ratio of rare cells to total cells is at most 5%, preferably at most 1%, especially at most 0.1%, such as at most 0.01 %. The method is particularly useful with rare cells, as the method increases the percentage of these cells considerably, which eases their detection or isolation. Rare cells may be in particular circulating tumor cells (CTC) and circulating tumor microemboli (CTM) in a patient's blood. The technical challenge in this field consists of finding 'rare' tumor cells (just a few CTCs mixed with the approximately 10 million leukocytes and 5 billion erythrocytes in 1 ml of blood) and being able to distinguish them from other cells, particularly epithelial non-tumor cells and leukocytes. However, these cells may be detected long before the tumor itself is detectable by standard means (and therefore a first diagnostic tool), which is evidently highly advantageous in the treatment of the cancerous diseases.

Evidently, the present disclosure is of particular interest for the isolation or detection of cells indicative of a particular state, such as a disease. Accordingly, the cells to be detected or isolated may be e.g. cardiovascular cells or vascular cells or vascular cells released by an inflammatory process, stem cells (e.g. cancerous stem cells), cells indicative of a minimal residual disease, cancer cells (e.g. leukemia cells) or bacterial cells, e.g. indicative of an infection. In this context, the method may be used for genotyping, diagnosis, prognosis, monitoring treatment etc.

Cancer cells are characterized by particular markers. Examples which may be mentioned are: especially oncogenes and tumor suppressor genes such as p53, genes of the ras family erb-B2, c-myc, mdm2, c-fos, DPC4, FAP, nm23, RET, WT1, and the like, LOHs, for example with regard to p53, DCC, APC, Rb and the like and also BRCA1 and BRCA2 in hereditary tumors, microsatellite instability of MSH2, MLH1, WT1 and the like; also tumorous RNAs such as CEA, cytokeratins, e. g. CK20, BCL-2, MUC1, in particular tumor-specific splice variants hereof, MAGE3, Muc18, tyrosinase, PSA,PSM, BA46, Mage-1 and the like, or else morphogenic RNAs such as maspin, hCG, GIP, motilin, hTG, SCCA-1, AR, ER, PR, various hormones and the like ; -- furthermore, especially RNAs and proteins which affect the metastasizing profile, i. e. the expression of molecules involved in angiogenesis, motility, adhesion and matrix degradation such as bFGF, bFGF-R, VEGF, VEGF-Rs, such as VEGF-R1 or VEGF-R2, E-cadherin, integrins, selectins, MMPs, TIMPs, SF, SF-R and the like, the cell cycle profile or proliferation profile, such as cyclins (e. g. expression ratio of cyclins D, E and B), Ki67, p120, p21, PCNA and the like, or the apoptosis profile, such as FAS (L+R), TNF (L+R), perforin, granzyme B, BAX, bcl-2, caspase 3 and the like. Accordingly, erythrocytes may be removed from a sample in order to increase concentration of other cells and allow for the detection of the above markers.

In a second aspect, the present disclosure relates to a method of lysing erythrocytes, the method comprising
a) providing a sample comprising erythrocytes; and
b) incubating the sample with the reagent for the lysis of erythrocytes, the reagent being an aqueous solution comprising or consisting of HEPES, NH₄⁺/NH₃ and a chelating agent, wherein the final concentration during lysis of erythrocytes is in the range of
   - from 2.5 mmol/l to 12 mmol/l HEPES,
   - from 60 mmol/l to 120 mmol/l NH₄⁺/NH₃, and
   - from 0.04 mmol/l to 0.8 mmol/l chelating agent,
wherein the chelating agent is selected from the group consisting of EGTA (ethylene glycol tetraacetic acid), BAPTA (1,2-bis(o-aminophenoxy)ethane-N,N,N',N'-tetraacetic acid), DTPA (diethylene triamine pentaacetic acid), EDTA (ethylenediamine tetraacetate) and NTA (N,N-bis(carboxymethyl)glycine).

The reagent may further comprise from 0.15 mmol/l to 0.8 mmol/l CO₃²⁻/CO₃⁻.

The method may further comprise after step b) optionally removing erythrocyte debris.

With respect to the terms used in the second aspect of the present disclosure it is referred to the terms, examples and specific embodiments used in the first aspect of the present disclosure, which are also applicable to the second aspect of the present disclosure.

As a first step of the method as presented herein a sample comprising erythrocytes is provided. Details on a suitable sample are given above. The sample may be contained in a vessel, wherein the vessel is a tube, such as a centrifuge tube or spin tube, syringes, cartridge, chamber, multiple-well plate, or test tube, or combinations thereof. The sample may be pre-treated in order to support lysis or erythrocytes or detecting or isolation or other cells. The size / volume of the sample may vary and may be chosen depending from the method to be carried out. If e.g. the method is used for the isolation of cells other than erythrocytes, the sample size will depend from the frequency of these cells.

In a second step the sample is incubated with the reagent as defined above, thereby lysing erythrocytes. For this, the sample may be added to the reagent or the reagent may be added to the sample. The result of contacting is an aqueous solution. The contacting is for a time and under conditions suitable for allowing the lysis of the erythrocytes.

Suitable conditions include appropriate temperature and solution to avoid e.g. death of cells other than erythrocytes or denaturation of proteins of interest, as far as present and required. Suitable conditions will depend from the particular design of the method and sample chosen and the skilled person will be able to select the same based on his general knowledge. Incubation steps can vary from about 5 seconds to several hours, preferably from about 5 minutes to about 30 hours. However, the incubation time will depend upon the method design, volume of solution, concentrations and the like. Usually, the methods will be carried out at ambient temperature, although they can be conducted over a range of temperatures, such as 20° C to 40° C or 22°C to 37°C. During contacting, the mixture of reagent and sample may be agitated, vortexed or shaken or may be left to stand.

As a third and optional step, the erythrocyte debris may be removed, i.e. separated from the remainder, e.g. cells of interest. Typically, cellular debris is removed by techniques involving differences physical characteristics of debris and remainder, such as sedimentation time and size. Typical methods include centrifugation and filtration. Methods for removal erythrocyte debris are well known in the art.

In a particular embodiment as presented herein, the second and/or third step may be repeated. Accordingly, the method may comprise one or more (e.g. two or three) lysis steps (i.e. step b)) and/or one or more (e.g. two or three) washing steps (i.e. step c)). In a very specific embodiment the method comprise twice step b) and one, two or three times step c).

In yet another specific embodiment, the sample is a blood sample or a sample comprising erythrocytes and other cells, particularly white blood cells and/or circulating tumor cells (see also above details).

In yet another specific embodiment, the method as presented herein further comprises c) detecting or isolating cells other than erythrocytes from a sample comprising erythrocytes, particularly from a blood sample, as described above.

For further details on detecting or isolating please see above.

Preferably, the cells other than erythrocytes are white blood cells or circulating tumor cells, particularly circulating tumor cells (see also above details).

Preferably, the incubating of step b) is for at most 30 min, preferably at most 20 min, more preferably for at most 10 min, especially at room temperature.

Also disclosed, but not part of the invention, is a kit for the isolation of white blood cells from a sample comprising erythrocytes, comprising
- a reagent for lysis of erythrocytes as defined above in the context of the uses and methods as disclosed herein; and
- a reagent for removing erythrocyte debris; and
- optionally, instructions for carrying out any of the methods as disclosed herein.

With respect to this disclosure it is referred to the terms, examples and specific embodiments used in the first and second aspect of the present disclosure, which are also applicable to the third aspect of the present disclosure.

The reagent for removing erythrocyte debris may be phosphate-buffered saline (PBS) comprising a chelating agent, especially in the range of from 0.1 mmol/l to 0.5 mmol/1, preferably in the range of from 0.2 mmol/l to 0.4 mmol/l, more preferably from 0.25 to 0.35 mmol/1, and/or especially wherein the chelating agent is EDTA.

PBS is a buffer solution commonly used in the biological, biochemical and medical field. It is a water-based salt solution comprising sodium chloride, sodium phosphate, and, in some formulations, potassium chloride and potassium phosphate. The buffer's phosphate groups help to maintain a constant pH. The osmolarity and ion concentrations of the solution usually match those of the human body (isotonic). PBS with EDTA is also used to disengage attached and clumped cells. Divalent metals such as zinc can be added to support precipitation. There are many different preparations of PBS. Some formulations do not contain potassium, while others contain calcium or magnesium. Generally, PBS comprises the following constituents (mmol/l): NaCl (137), KCl (2.7), Na₂HPO₄ (10), KH₂PO₄ (2.0). The pH is usually about 7.4. If used with cells, the solution can be dispensed into aliquots and sterilized by autoclaving (20 min, 121°C, liquid cycle). Sterilization may not be necessary depending on its use. PBS can be stored at room temperature. However, concentrated stock solutions may precipitate when cooled and should be kept at room temperature until precipitate has completely dissolved before use. In the context of the present disclosure PBS comprises (mmol/l) e.g. NaCl (138), KCl (2.7), Na₂HPO₄ (8), KH₂PO₄ (1.5) and has a pH of 7.0 to 7.6, preferably 7.2 to 7.4.

The terminology used herein is for the purpose of describing particular embodiments only and is not intended to limit the scope of the present disclosure. The words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the disclosure. Although any methods and materials similar or equivalent to those described herein can be used in the practice as presented herein, the specific methods, and materials are described herein.

The disclosure is further illustrated by the following examples, although it will be understood that the examples are included merely for purposes of illustration.

### EXAMPLES

In order to test the various reagents (referred to as lysis buffer) in the lysis of erythrocytes, while maintaining other cells, the following protocol was used:
Unless otherwise noted, one part of whole blood is mixed with parts of the lysis buffer (as defined below), incubated for 10 min at room temperature and centrifugated for 15 min at 300 x g. The supernatant is discarded and cell pellet is resuspended in four parts of the lysis buffer and centrifugated for 15 min at 300 x g. Supernatant is discarded and cell pellet is resuspended in four parts of PBS containing 0.3 mM EDTA and centrifugated for 15 min at 300 x g. The supernatant is discarded and cell pellet is resuspended in a distinct amount of PBS containing 0.3 mM EDTA.

### Comparative Example 1: Erythrocyte Lysis with Conventional Products

In a first test, the suitability of conventional products was tested according to the above protocol. The following conventional products were used EasySep^{®} Red Blood Cell Lysis Buffer (StemCell Technologies, Cat. No. 20110), HetaSep^{®} (StemCell Technologies, Cat. No. 07806), Stromatolyser NR Lyse (Sysmex, Cat. No. SNR-200, SNR-210A).

The results are shown in following table 1:

**Table 1: Effect of Conventional Products for Erythrocyte Lysis on Recovery Rate of White Blood Cells (WBCs)**

| **Easysep 1 portion blood + 2 portions lysis buffer, 2 × lysis (10 min), 2 × washing steps** | | | | |
|---|---|---|---|---|
| WBCs/µl Blood | WBCs/µl | % | Mean % | SD |
| 6300 | 5600 | 88.89 | | |
| | 5700 | 90.48 | | |
| | 4900 | 77.78 | 85.71 | 6.92 |

| **HetaSep 5 portions blood + 1 portion lysis buffer, 1 × lysis (11 min), 2 × washing steps** | | | | |
|---|---|---|---|---|
| WBCs/µl Blood | WBCs/µl | % | Mean % | SD |
| 6500 | 3200 | 49.23 | | |
| | 2900 | 44.62 | | |
| | 3200 | 49.23 | 47.69 | 2.66 |

| **Stromatolyser (Sysmex) 1 portion blood + 2 portions lysis buffer, 1 × lysis (ca. 30 sec), 2 × washing steps** | | | | |
|---|---|---|---|---|
| WBCs/µl Blood | WBCs/µl | % | Mean % | SD |
| 6500 | 500 | 7.69 | | |
| | 600 | 9.23 | 8.46 | 1.09 |

| **Stromatolyser (Sysmex) 1 portion blood + 5 portions lysis buffer, 1 × lysis (ca. 30 sec), 2 × washing steps** | | | | |
|---|---|---|---|---|
| WBCs/µl Blood | WBCs/µl | % | Mean % | SD |
| 6500 | 4000 | 61.54 | | |
| | 4200 | 64.62 | 63.08 | 2.18 |

The above results show that with conventional products a high percentage of white blood cells (WBC) is lost during the lysis of erythrocytes, namely up to more than 50 %.

### Comparative Example 2: Erythrocyte Lysis in reagent without NH₄Cl

In a second test, the suitability of reagents without NH₄Cl was tested according to the above protocol. The results are shown in following table 2:

**Table 2: Effect of reagent without NH₄Cl on Recovery of White Blood Cells (WBCs)**

| **Lysis buffer: 0.225 % NaCl,** | | | | |
|---|---|---|---|---|
| **1 portion blood + 5 portions lysis buffer, 2 × lysis (10 min), 2 × washing steps** | | | | |
| WBCS/µl blood | WBCS/µl | % | Mean % | SD |
| 5900 | 3900 | 66.10 | | |
| | 3600 | 61.02 | | |
| | 3400 | 57.63 | 61.58 | 4.27 |

| **Lysis buffer: 0.225 % NaCl,** | | | | |
|---|---|---|---|---|
| **1 portion blood + 9 portions lysis buffer, 2 × lysis (10 min), 2 × washing steps** | | | | |
| WBCS/µl blood | WBCS/µl | % | Mean % | SD |
| 5900 | 3000 | 50.85 | | |
| | 2900 | 49.15 | | |
| | 2600 | 44.07 | 48.02 | 3.53 |

| **Lysis buffer: 0.3 % HAc, 45 mM Na₂CO₃** | | | | |
|---|---|---|---|---|
| **1 portion blood + 10.5 portions lysis buffer, 2 × washing steps** | | | | |
| WBCS/µl blood | WBCS/µl | % | Mean % | SD |
| 5300 | 4500 | 84.91 | | |
| | 4400 | 83.02 | | |
| | 4000 | 75.47 | 81.13 | 4.99 |
| 9000 | 7400 | 82.22 | | |
| | 7600 | 84.44 | | |
| | 4000 | 44.44 | 70.37 | 22.48 |

The above results show that buffers without NH₄Cl are not suitable for the intended use, as a considerable portion of white blood cells (WBC) is lost during the lysis of erythrocytes.

### Comparative Example 3: Erythrocyte Lysis in different reagents with NH₄Cl

In a third test, the suitability of different reagents with NH₄Cl was tested according to the above protocol. The results are shown in following table 3:

**Table 3: Effect of different reagents with NH₄Cl on Recovery of White Blood Cells (WBCs)**

| **Lysis buffer: 150 mM NH₄Cl + 10 mM NaAc, pH 5** | | | | |
|---|---|---|---|---|
| **1 portion blood + 5 portions lysis buffer, 2 × lysis (10 min), 2 × washing steps** | | | | |
| WBCS/µl Blood | WBCS/µl | % | Mean % | SD |
| 11400 | 10500 | 92.11 | | |
| | 9500 | 83.33 | | |
| | 9600 | 84.21 | 86.55 | 4.83 |

| **Lysis buffer: 75 mM NH₄Cl + 0.45 % NaCl** | | | | |
|---|---|---|---|---|
| **1 portion blood + 5 portions lysis buffer, 2 × lysis (10 min), 2 × washing steps** | | | | |
| WBCS/µl Blood | WBCS/µl | % | Mean % | SD |
| 11400 | 6700 | 58.77 | | |
| | 7600 | 66.67 | | |
| | 6400 | 56.14 | 60.53 | 5.48 |

| **Lysis buffer: 150 mM NH₄Cl + 10 mM Tris pH 7.5** | | | | |
|---|---|---|---|---|
| **1 portion blood + 5 portions lysis buffer, 2 × lysis (10 min), 2 × washing steps** | | | | |
| WBCS/µl Blood | WBCS/µl | % | Mean % | SD |
| 5300 | 4900 | 92.45 | | |
| | 4100 | 77.36 | | |
| | 4600 | 86.79 | 85.53 | 7.63 |

| **Lysis buffer: 150 mM NH₄Cl + 5 mM Hepes** | | | | |
|---|---|---|---|---|
| **1 portion blood + 6 portions lysis buffer, 1 × lysis (10 min), 2 × washing steps** | | | | |
| WBCs/µl Blood | WBCs/µl | % | Mean % | SD % |
| 9000 | 8400 | 93.33 | | |
| | 7900 | 87.78 | | |
| | 7900 | 87.78 | 89.63 | 3.21 |
| 8400 | 7600 | 90.48 | | |
| | 7100 | 84.52 | | |
| | 7100 | 84.52 | 86.51 | 3.44 |

| **Lysis buffer: 80 mM NH₄Cl + 10 mM Tris pH 7.5** | | | | |
|---|---|---|---|---|
| **1 portion blood + 5 portions lysis buffer, 1 × lysis (10 min)** | | | | |
| WBCs/µl Blood | WBCs/µl | % | Mean % | SD % |
| 10700 | 8700 | 81.31 | | |
| | 9200 | 85.98 | 83.64 | 3.30 |
| 5100 | 4300 | 84.31 | | |
| | 4400 | 86.27 | 85.29 | 1.39 |
| 5500 | 4100 | 74.55 | | |
| | 4600 | 83.64 | 79.09 | 6.43 |
| 7100 | 5400 | 76.06 | | |
| | 5800 | 81.69 | 78.87 | 3.98 |

| **Lysis buffer: 80 mM NH₄Cl + 10 mM Tris pH 7.5 +0.1 mM EDTA** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 10700 | 9200 | 85.98 | | |
| | 8800 | 82.24 | 84.11 | 2.64 |
| 5100 | 4600 | 90.20 | | |
| | 4600 | 90.20 | 90.20 | 0.00 |
| 3200 | 2600 | 81.25 | | |
| | 2700 | 84.38 | 82.81 | 2.21 |
| 7400 | 6200 | 83.78 | | |
| | 7000 | 94.59 | 89.19 | 7.64 |

The above results show that buffers with NH₄Cl and HEPES are suitable for the intended use, as only a low number of white blood cells (WBC) is lost during the lysis of erythrocytes. Additionally, EDTA seems to increase WBCs recovery.

### Example 4 (comprising both comparative examples and an embodiment falling within the scope of the present invention): Erythrocyte Lysis in different reagents with NH₄Cl and HEPES

In a forth test, the suitability of different reagents with NH₄Cl and HEPES was tested according to the above protocol. The results are shown in following table 4:

**Table 4: Effect of different reagents with NH₄Cl and HEPES on Recovery of White Blood Cells (WBCs)**

| **Lysis buffer: 80 mM NH₄Cl + 5 mM HEPES** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 4700 | 4200 | 89.36 | | |
| | 3600 | 76.60 | 82.98 | 9.03 |
| 5300 | 4900 | 92.45 | | |
| | 3800 | 71.70 | 82.08 | 14.68 |
| 6900 | 5900 | 85.51 | | |
| | 5600 | 81.16 | 83.33 | 3.07 |
| 5700 | 5100 | 89.47 | | |
| | 4700 | 82.46 | 85.96 | 4.96 |
| 4200 | 3400 | 80.95 | | |
| | 3400 | 80.95 | 80.95 | 0.00 |
| 6200 | 4900 | 79.03 | | |
| | 5000 | 80.65 | 79.84 | 1.14 |
| 5700 | 4800 | 84.21 | | |
| | 5100 | 89.47 | 86.84 | 3.72 |

| **Lysis buffer: 80 mM NH₄Cl + 10 mM HEPES** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 8800 | 7600 | 86.36 | | |
| | 7900 | 89.77 | 88.07 | 2.41 |
| 4700 | 3700 | 78.72 | | |
| | 3800 | 80.85 | 79.79 | 1.50 |
| 7100 | 6200 | 87.32 | | |
| | 6500 | 91.55 | 89.44 | 2.99 |
| 5800 | 5200 | 89.66 | | |
| | 5300 | 91.38 | 90.52 | 1.22 |
| 5000 | 4100 | 82.00 | | |
| | 4500 | 90.00 | 86.00 | 5.66 |
| 5700 | 5000 | 87.72 | | |
| | 5700 | 100.00 | 93.86 | 8.68 |
| 5600 | 5000 | 89.29 | | |
| | 4900 | 87.50 | 88.39 | 1.26 |
| 6100 | 5100 | 83.61 | | |
| | 5400 | 88.52 | 86.07 | 3.48 |
| 5500 | 4800 | 87.27 | | |
| | 4900 | 89.09 | 88.18 | 1.29 |
| 7100 | 5500 | 77.46 | | |

| **Lysis buffer: 80 mM NH₄Cl + 5 mM HEPES + 0.5 mM KHCO₃ + 0.1 mM EDTA** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 5600 | 5000 | 89.29 | | |
| | 5200 | 92.86 | 91.07 | 2.53 |
| 6100 | 5500 | 90.16 | | |
| | 5200 | 85.25 | 87.70 | 3.48 |
| 3200 | 2800 | 87.50 | | |
| | 2800 | 87.50 | 87.50 | 0.00 |
| 7400 | 6800 | 91.89 | | |
| | 6700 | 90.54 | 91.22 | 0.96 |
| 6700 | 5600 | 83.58 | | |
| | 5900 | 88.06 | | |
| | 5900 | 88.06 | | |
| | 5800 | 86.57 | 86.57 | 2.11 |
| 5600 | 4600 | 82.14 | | |
| | 4600 | 82.14 | | |
| | 4500 | 80.36 | | |
| | 4700 | 83.93 | 82.14 | 1.46 |
| 6600 | 5500 | 83.33 | | |
| | 5700 | 86.36 | | |
| | 5500 | 83.33 | | |
| | 5800 | 87.88 | 85.23 | 2.27 |
| 3800 | 3100 | 81.58 | | |
| | 3000 | 78.95 | | |
| | 3100 | 81.58 | | |
| | 3100 | 81.58 | 80.92 | 1.32 |
| 6800 | 6400 | 94.12 | | |
| | 6000 | 88.24 | | |
| | 6400 | 94.12 | | |
| | 6200 | 91.18 | 91.91 | 2.82 |
| 4700 | 4400 | 93.62 | | |
| | 4400 | 93.62 | | |
| | 4200 | 89.36 | | |
| | 4300 | 91.49 | 92.02 | 2.04 |
| 7300 | 6500 | 89.04 | | |
| | 7000 | 95.89 | | |
| | 6700 | 91.78 | | |
| | 6900 | 94.52 | 92.81 | 3.04 |
| 7200 | 6800 | 94.44 | | |
| | 6500 | 90.28 | | |
| | 6200 | 86.11 | | |
| | 6600 | 91.67 | 90.63 | 3.47 |
| 6900 | 6700 | 97.10 | | |
| | 6700 | 97.10 | | |
| | 6300 | 91.30 | | |
| | 6400 | 92.75 | 94.57 | 2.99 |
| 5000 | 4300 | 86.00 | | |
| | 4600 | 92.00 | | |
| | 4500 | 90.00 | | |
| | 4800 | 96.00 | 91.00 | 4.16 |
| 6800 | 6000 | 88.24 | | |
| | 6100 | 89.71 | | |
| | 5700 | 83.82 | | |
| | 6500 | 95.59 | 89.34 | 4.86 |
| 4800 | 4300 | 89.58 | | |
| | 4500 | 93.75 | | |
| | 4400 | 91.67 | | |
| | 4400 | 91.67 | 91.67 | 1.70 |
| 4300 | 3900 | 90.70 | | |
| | 4000 | 93.02 | | |
| | 3900 | 90.70 | | |
| | 3900 | 90.70 | 91.28 | 1.16 |
| 6900 | 6200 | 89.86 | | |
| | 6500 | 94.20 | | |
| | 6500 | 94.20 | | |
| | 6500 | 94.20 | 93.12 | 2.17 |
| 5900 | 5100 | 86.44 | | |
| | 5200 | 88.14 | | |
| | 5400 | 91.53 | | |
| | 5300 | 89.83 | 88.98 | 2.19 |
| 5800 | 4800 | 82.76 | | |
| | 5500 | 94.83 | | |
| | 5400 | 93.10 | | |
| | 5400 | 93.10 | 90.95 | 5.52 |
| 12900 | 11800 | 91.47 | | |
| | 12000 | 93.02 | | |
| | 12400 | 96.12 | | |
| | 12500 | 96.90 | 94.38 | 2.56 |
| 7400 | 6500 | 87.84 | | |
| | 7400 | 100.00 | | |
| | 6800 | 91.89 | | |
| | 6700 | 90.54 | 92.57 | 5.23 |
| 6600 | 5700 | 86.36 | | |
| | 5600 | 84.85 | | |
| | 6100 | 92.42 | 87.88 | 4.01 |
| 6300 | 5300 | 84.13 | | |
| | 5800 | 92.06 | | |
| | 5400 | 85.71 | | |
| | 5800 | 92.06 | 88.49 | 4.17 |
| 7000 | 6000 | 85.71 | | |
| | 6100 | 87.14 | | |
| | 6000 | 85.71 | | |
| | 6100 | 87.14 | 86.43 | 0.82 |
| 4600 | 3800 | 82.61 | | |
| | 4000 | 86.96 | | |
| | 4200 | 91.30 | | |
| | 4100 | 89.13 | 87.50 | 3.71 |
| 5100 | 4500 | 88.24 | | |
| | 4400 | 86.27 | | |
| | 4300 | 84.31 | | |
| | 4200 | 82.35 | 85.29 | 2.53 |
| 5600 | 4700 | 83.93 | | |
| | 4600 | 82.14 | | |
| | 4400 | 78.57 | 81.55 | 2.73 |
| 8500 | 7900 | 92.94 | | |
| | 7600 | 89.41 | | |
| | 7400 | 87.06 | 89.80 | 2.96 |
| 4500 | 3800 | 84.44 | | |
| | 4300 | 95.56 | | |
| | 3900 | 86.67 | 88.89 | 5.88 |
| 3400 | 3000 | 88.24 | | |
| | 3000 | 88.24 | | |
| | 2900 | 85.29 | 87.25 | 1.70 |
| 5400 | 4300 | 79.63 | | |
| | 4700 | 87.04 | | |
| | 4800 | 88.89 | 85.19 | 4.90 |
| 6200 | 5300 | 85.48 | | |
| | 5700 | 91.94 | | |
| | 5600 | 90.32 | 89.25 | 3.36 |
| 4600 | 3900 | 84.78 | | |
| | 4400 | 95.65 | | |
| | 4200 | 91.30 | 90.58 | 5.47 |
| 5900 | 5100 | 86.44 | | |
| | 5200 | 88.14 | | |
| | 5400 | 91.53 | 88.70 | 2.59 |
| 8900 | 7500 | 84.27 | | |
| | 7600 | 85.39 | | |
| | 7500 | 84.27 | 84.64 | 0.65 |
| 10000 | 9200 | 92.00 | | |
| | 8900 | 89.00 | | |
| | 9000 | 90.00 | 90.33 | 1.53 |
| 6500 | 6000 | 92.31 | | |
| | 5600 | 86.15 | | |
| | 5700 | 87.69 | 88.72 | 3.20 |
| 11500 | 11000 | 95.65 | | |
| | 11100 | 96.52 | | |
| | 11000 | 95.65 | 95.94 | 0.50 |
| 11100 | 10600 | 95.50 | | |
| | 10300 | 92.79 | | |
| | 11000 | 99.10 | 95.80 | 3.16 |
| 7500 | 7200 | 96.00 | | |
| | 7100 | 94.67 | | |
| | 7100 | 94.67 | 95.11 | 0.77 |
| 6000 | 5300 | 88.33 | | |
| | 5500 | 91.67 | | |
| | 5300 | 88.33 | 89.44 | 1.92 |
| 4300 | 4400 | 102.33 | | |
| | 4100 | 95.35 | | |
| | 4100 | 95.35 | 97.67 | 4.03 |
| 7700 | 6900 | 89.61 | | |
| | 7800 | 101.30 | | |
| | 7700 | 100.00 | 96.97 | 6.41 |
| 5200 | 4600 | 88.46 | | |
| | 4600 | 88.46 | | |
| | 4800 | 92.31 | 89.74 | 2.22 |
| 6400 | 6300 | 98.44 | | |
| | 6100 | 95.31 | | |
| | 5400 | 84.38 | 92.71 | 7.38 |
| 7000 | 6800 | 97.14 | | |
| | 6500 | 92.86 | | |
| | 6200 | 88.57 | 92.86 | 4.29 |
| 4800 | 4300 | 89.58 | | |
| | 3800 | 79.17 | | |
| | 3800 | 79.17 | 82.64 | 6.01 |
| 6400 | 6000 | 93.75 | | |
| | 5600 | 87.50 | | |
| | 5800 | 90.63 | 90.63 | 3.13 |
| 9900 | 9300 | 93.94 | | |
| | 8600 | 86.87 | | |
| | 8900 | 89.90 | 90.24 | 3.55 |
| 5500 | 4700 | 85.45 | | |
| | 5200 | 94.55 | | |
| | 4900 | 89.09 | 89.70 | 4.58 |
| 6400 | 5700 | 89.06 | | |
| | 6000 | 93.75 | | |
| | 5600 | 87.50 | 90.10 | 3.25 |
| 5000 | 4700 | 94.00 | | |
| | 4400 | 88.00 | | |
| | 4500 | 90.00 | 90.67 | 3.06 |
| 6300 | 5500 | 87.30 | | |
| | 5500 | 87.30 | | |
| | 5200 | 82.54 | 85.71 | 2.75 |
| 6700 | 6300 | 94.03 | | |
| | 6500 | 97.01 | | |
| | 6000 | 89.55 | 93.53 | 3.76 |
| 6100 | 5100 | 83.61 | | |
| | 5400 | 88.52 | | |
| | 5600 | 91.80 | 87.98 | 4.13 |
| 5100 | 4300 | 84.31 | | |
| | 4200 | 82.35 | | |
| | 4400 | 86.27 | 84.31 | 1.96 |
| 6900 | 6100 | 88.41 | | |
| | 6800 | 98.55 | | |
| | 6700 | 97.10 | 94.69 | 5.49 |
| 10300 | 10000 | 97.09 | | |
| | 9700 | 94.17 | | |
| | 10300 | 100.00 | 97.09 | 2.91 |
| 8100 | 7400 | 91.36 | | |
| | 8200 | 101.23 | | |
| | 8200 | 101.23 | 97.94 | 5.70 |
| 4900 | 4300 | 87.76 | | |
| | 4300 | 87.76 | | |
| | 4700 | 95.92 | 90.48 | 4.71 |
| 6200 | 5500 | 88.71 | | |
| | 5500 | 88.71 | | |
| | 5500 | 88.71 | 88.71 | 0.00 |
| 4800 | 4500 | 93.75 | | |
| | 4400 | 91.67 | | |
| | 4700 | 97.92 | 94.44 | 3.18 |
| 5800 | 5600 | 96.55 | | |
| | 5700 | 98.28 | | |
| | 5500 | 94.83 | 96.55 | 1.72 |
| 4200 | 4000 | 95.24 | | |
| | 3800 | 90.48 | | |
| | 4200 | 100.00 | 95.24 | 4.76 |
| 7900 | 7700 | 97.47 | | |
| | 7700 | 97.47 | | |
| | 7700 | 97.47 | 97.47 | 0.00 |
| 6800 | 6200 | 91.18 | | |
| | 6000 | 88.24 | | |
| | 6600 | 97.06 | 92.16 | 4.49 |
| 8200 | 7100 | 86.59 | | |
| | 7400 | 90.24 | | |
| | 7800 | 95.12 | 90.65 | 4.28 |
| 9900 | 8900 | 89.90 | | |
| | 9600 | 96.97 | | |
| | 9600 | 96.97 | 94.61 | 4.08 |
| 6100 | 5500 | 90.16 | | |
| | 5200 | 85.25 | | |
| | 5400 | 88.52 | 87.98 | 2.50 |
| 7500 | 6500 | 86.67 | | |
| | 6200 | 82.67 | | |
| | 6400 | 85.33 | 84.89 | 2.04 |
| 6800 | 6100 | 89.71 | | |
| | 5900 | 86.76 | | |
| | 5900 | 86.76 | 87.75 | 1.70 |
| 5700 | 4900 | 85.96 | | |
| | 5000 | 87.72 | | |
| | 4800 | 84.21 | 85.96 | 1.75 |
| 8200 | 7200 | 87.80 | | |
| | 7400 | 90.24 | | |
| | 7000 | 85.37 | 87.80 | 2.44 |
| 5700 | 4600 | 80.70 | | |
| | 4900 | 85.96 | | |
| | 4800 | 84.21 | 83.63 | 2.68 |
| 6700 | 6000 | 89.55 | | |
| | 5500 | 82.09 | | |
| | 5600 | 83.58 | 85.07 | 3.95 |
| 3400 | 2600 | 76.47 | | |
| | 2900 | 85.29 | | |
| | 2700 | 79.41 | 80.39 | 4.49 |
| 13000 | 13000 | 100.00 | | |
| | 13600 | 104.62 | | |
| | 14000 | 107.69 | 104.10 | 3.87 |
| 5400 | 4800 | 88.89 | | |
| | 5000 | 92.59 | | |
| | 5200 | 96.30 | 92.59 | 3.70 |
| 8500 | 7900 | 92.94 | | |
| | 7800 | 91.76 | | |
| | 8500 | 100.00 | 94.90 | 4.45 |
| 6300 | 5300 | 84.13 | | |
| | 5700 | 90.48 | | |
| | 5200 | 82.54 | 85.71 | 4.20 |
| 6800 | 6400 | 94.12 | | |
| | 5700 | 83.82 | | |
| | 6300 | 92.65 | 90.20 | 5.57 |
| 5700 | 5400 | 94.74 | | |
| | 5400 | 94.74 | | |
| | 5400 | 94.74 | 94.74 | 0.00 |
| 9400 | 8500 | 90.43 | | |
| | 8800 | 93.62 | | |
| | 8700 | 92.55 | 92.20 | 1.63 |
| 5000 | 4000 | 80.00 | | |
| | 4000 | 80.00 | | |
| | 4500 | 90.00 | 83.33 | 5.77 |
| 6100 | 5500 | 90.16 | | |
| | 5400 | 88.52 | | |
| | 5600 | 91.80 | 90.16 | 1.64 |
| 4900 | 4400 | 89.80 | | |
| | 4300 | 87.76 | | |
| | 4000 | 81.63 | 86.39 | 4.25 |
| 13300 | 11600 | 87.22 | | |
| | 12400 | 93.23 | | |
| | 12600 | 94.74 | 91.73 | 3.98 |
| 4200 | 4100 | 97.62 | | |
| | 3800 | 90.48 | | |
| | 4000 | 95.24 | 94.44 | 3.64 |
| 12800 | 11600 | 90.63 | | |
| | 12500 | 97.66 | | |
| | 12200 | 95.31 | 94.53 | 3.58 |
| 6400 | 5900 | 92.19 | | |
| | 6100 | 95.31 | | |
| | 6300 | 98.44 | 95.31 | 3.13 |
| 4700 | 4300 | 91.49 | | |
| | 4100 | 87.23 | | |
| | 4300 | 91.49 | 90.07 | 2.46 |
| 6500 | 6200 | 95.38 | | |
| | 6100 | 93.85 | | |
| | 6700 | 103.08 | 97.44 | 4.95 |
| 5000 | 4600 | 92.00 | | |
| | 5100 | 102.00 | | |
| | 5000 | 100.00 | 98.00 | 5.29 |
| 6800 | 6400 | 94.12 | | |
| | 6500 | 95.59 | | |
| | 6500 | 95.59 | 95.10 | 0.85 |
| 7800 | 7700 | 98.72 | | |
| | 7200 | 92.31 | | |
| | 7700 | 98.72 | 96.58 | 3.70 |
| 5500 | 5500 | 100.00 | | |
| | 5500 | 100.00 | | |
| | 5700 | 103.64 | 101.21 | 2.10 |
| 9300 | 9300 | 100.00 | | |
| | 9600 | 103.23 | | |
| | 9500 | 102.15 | 101.79 | 1.64 |
| 6300 | 5400 | 85.71 | | |
| | 5900 | 93.65 | | |
| | 6300 | 100.00 | 93.12 | 7.16 |
| 6500 | 5500 | 84.62 | | |
| | 6000 | 92.31 | | |
| | 6800 | 104.62 | 93.85 | 10.09 |
| 6200 | 5900 | 95.16 | | |
| | 6000 | 96.77 | | |
| | 6300 | 101.61 | 97.85 | 3.36 |
| 4200 | 3800 | 90.48 | | |
| | 3800 | 90.48 | | |
| | 3800 | 90.48 | 90.48 | 0.00 |
| 7400 | 6900 | 93.24 | | |
| | 7300 | 98.65 | | |
| | 7200 | 97.30 | 96.40 | 2.81 |
| 4900 | 4700 | 95.92 | | |
| | 4400 | 89.80 | | |
| | 4600 | 93.88 | 93.20 | 3.12 |
| 8400 | 7400 | 88.10 | | |
| | 7800 | 92.86 | | |
| | 8300 | 98.81 | 93.25 | 5.37 |
| 5800 | 5200 | 89.66 | | |
| | 5400 | 93.10 | | |
| | 5700 | 98.28 | 93.68 | 4.34 |
| 7800 | 6700 | 85.90 | | |
| | 6700 | 85.90 | | |
| | 7100 | 91.03 | 87.61 | 2.96 |
| 3900 | 3300 | 84.62 | | |
| | 3500 | 89.74 | | |
| | 3500 | 89.74 | 88.03 | 2.96 |
| 5200 | 4200 | 80.77 | | |
| | 4800 | 92.31 | | |
| | 5100 | 98.08 | 90.38 | 8.81 |
| 6600 | 5900 | 89.39 | | |
| | 6000 | 90.91 | | |
| | 6200 | 93.94 | 91.41 | 2.31 |
| 5400 | 5000 | 92.59 | | |
| | 4900 | 90.74 | | |
| | 4800 | 88.89 | 90.74 | 1.85 |
| 4400 | 3200 | 72.73 | | |
| | 3600 | 81.82 | | |
| | 3200 | 72.73 | 75.76 | 5.25 |
| 5700 | 5200 | 91.23 | | |
| | 5300 | 92.98 | | |
| | 4800 | 84.21 | 89.47 | 4.64 |
| 5800 | 4400 | 75.86 | | |
| | 4800 | 82.76 | | |
| | 5000 | 86.21 | 81.61 | 5.27 |
| 7700 | 7400 | 96.10 | | |
| | 7500 | 97.40 | | |
| | 7600 | 98.70 | 97.40 | 1.30 |
| 6300 | 5800 | 92.06 | | |
| | 5400 | 85.71 | | |
| | 5800 | 92.06 | 89.95 | 3.67 |
| 6100 | 6200 | 101.64 | | |
| | 6700 | 109.84 | | |
| | 6500 | 106.56 | 106.01 | 4.13 |
| 5900 | 4500 | 76.27 | | |
| | 4900 | 83.05 | | |
| | 5500 | 93.22 | 84.18 | 8.53 |
| 6700 | 5900 | 88.06 | | |
| | 5800 | 86.57 | | |
| | 6000 | 89.55 | 88.06 | 1.49 |
| 6800 | 6000 | 88.24 | | |
| | 6100 | 89.71 | | |
| | 6600 | 97.06 | 91.67 | 4.73 |
| 8300 | 8300 | 100.00 | | |
| | 7500 | 90.36 | | |
| | 7300 | 87.95 | 92.77 | 6.38 |
| 4300 | 3500 | 81.40 | | |
| | 3500 | 81.40 | | |
| | 3700 | 86.05 | 82.95 | 2.69 |

| **Lysis buffer: 80 mM NH₄Cl (based on RBCL buffer*) + 5 mM HEPES** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 6300 | 5500 | 87.30 | | |
| | 5400 | 85.71 | 86.51 | 1.12 |
| 9100 | 8600 | 94.51 | | |
| | 8500 | 93.41 | 93.96 | 0.78 |
| 6200 | 5500 | 88.71 | | |
| | 5200 | 83.87 | 86.29 | 3.42 |
| 5700 | 5100 | 89.47 | | |
| | 5200 | 91.23 | 90.35 | 1.24 |

| **Lysis buffer: 80 mM NH₄Cl (based on RBCL buffer*) + 10 mM HEPES** | | | | |
|---|---|---|---|---|
| **1 portion blood + 5 portions lysis buffer, 2 × lysis (10 min)** | | | | |
| WBCs/µl Blood | WBCs/µl | % | Mean % | SD % |
| 6300 | 5800 | 92.06 | | |
| | 5600 | 88.89 | 90.48 | 2.24 |
| 9100 | 8500 | 93.41 | | |
| | 8400 | 92.31 | 92.86 | 0.78 |
| 8800 | 8300 | 94.32 | | |
| | 8500 | 96.59 | 95.45 | 1.61 |
| 4700 | 4400 | 93.62 | | |
| | 4200 | 89.36 | 91.49 | 3.01 |

| | | | | |
|---|---|---|---|---|
| ^{∗}RBCL buffer- Red Blood Cell Lysis buffer (Roche Applied Science, Cat. No. 11814389001): 150 mM NH₄Cl, 1 mM KHCO₃, 0.1 mM | | | | |

Again, the above results show that buffers with NH₄Cl and HEPES are suitable for the intended use, as only a low number of white blood cells (WBC) is lost during the lysis of erythrocytes. Additionally, EDTA and KHCO₃ increase WBCs recovery. In the absence of these, 10 mM HEPES is superior to 5 mM HEPES.

For lysis buffer consisting of 80 mM NH₄Cl + 5 mM HEPES + 0.5 mM KHCO₃ + 0.1 mM EDTA (used with 1 portion blood + 5 portions lysis buffer, 2 × lysis (10 min)), 122 samples were tested. 90.77 ± 3.37 % oft he WBC present before lysis (100.00 ± 3.38 %) were recovered after lysis.

### Example 5: Erythrocyte Lysis in different reagents with NH₄Cl and HEPES

In a fifth test, the suitability of a reagent with 80 mM NH4Cl+ 10 mM Hepes + 0.1 mM EDTA was tested according to the above protocol. In addition to recovery of WBCs and RBCs, the viability of recovered WBCs was measured by Trypanblue exclusion test. Therefore the WBCs have been stained with Trypanblue (Sigma, Cat. No. T8154-20ML), (Dilution WBC suspension : Trypanblue = 1:2) and stained cells (dead cells) have been counted using the C-Chip counting chamber (Biochrom, Cat. No. P DHC-N01). The results are shown in following table 5:

**Table 5: Effect of reagent on Recovery and Viability of White Blood Cells (WBCs) and Red Blood Cells (RBCs)**

| **WBCs/ µl Blood** | **WBCs/µl** | **%** | **Mean %** | **SD %** | **non viable cells (%)** | **RBC/ µl Blood** | **RBC/µl** |
|---|---|---|---|---|---|---|---|
| 7100 | 6200 | 87.32 | 89.44 | 2.99 | 330 | 4.49×10⁶ | 0.04×10⁶ |
| | 6500 | 91.55 | | | (5.32) | | 0.04x10⁶ |
| 5800 | 5200 | 89.66 | 90.52 | 1.22 | 335 | 5.45×10⁶ | 0.04x10⁶ |
| | 5300 | 91.38 | | | (6.44) | | 0.03×10⁶ |
| 5000 | 4100 | 82.00 | 86.00 | 5.66 | 160 | 4.70×10⁶ | 0.02×10⁶ |
| | 4500 | 90.00 | | | (3.9) | | 0.03×10⁶ |
| 5700 | 5000 | 87.72 | 93.86 | 8.68 | 485 | 4.63×10⁶ | 0.03×10⁶ |
| | 5700 | 100.00 | | | (9.7) | | 0.04x10⁶ |
| 8800 | 8300 | 94.32 | 95.45 | 1.61 | 115 | 4.97×10⁶ | 0.03×10⁶ |
| | 8500 | 96.59 | | | (1.39) | | 0.02×10⁶ |
| 4700 | 4400 | 93.62 | 91.49 | 3.01 | 145 | 4.87×10⁶ | 0.03×10⁶ |
| | 4200 | 89.36 | | | (3.3) | | 0.02×10⁶ |

The above results show that while the number of RBCs is decreased drastically, the number and viability of WBCs is maintained at a very high level, proving the suitability of the reagent for the specific lysis of erythrocytes.

### Example 6: Erythrocyte Lysis in different reagents with NH₄Cl and HEPES

In a sixth test, the suitability of different reagents with NH₄Cl and HEPES was tested according to the above protocol. The results are shown in following table 6:

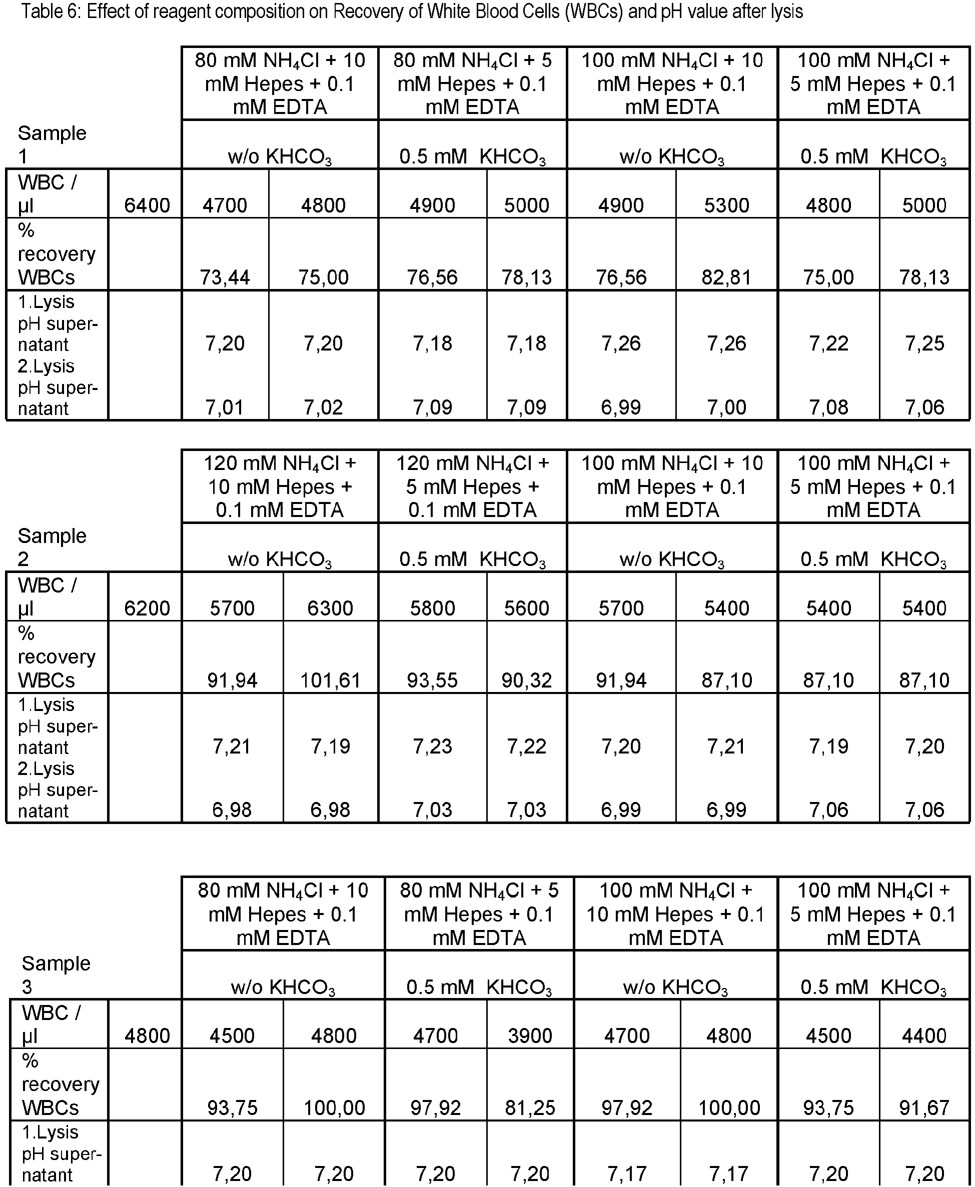

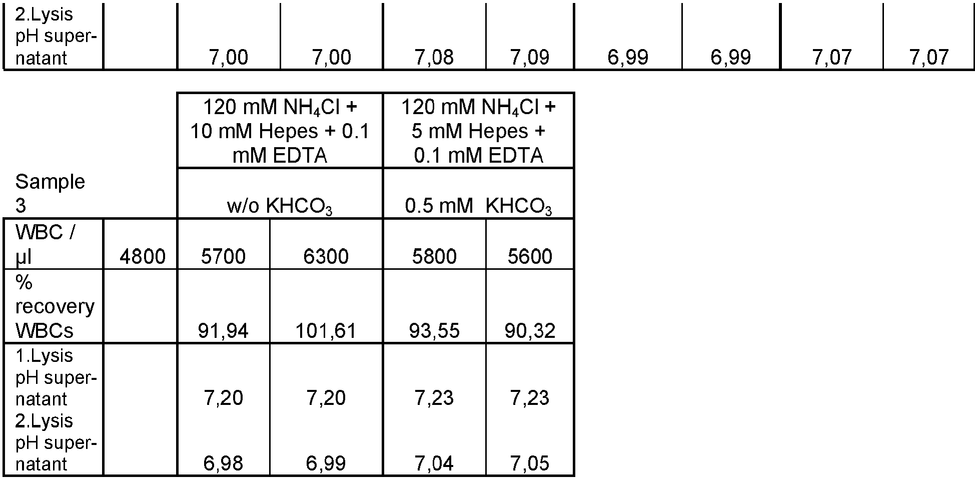

Various buffers with 80 to 120 mmol NH₄Cl + 5 mM to 10 mmol/l HEPES + 0.1 mM EDTA +0 to 0.5 mM KHCO₃ were tested for their effectiveness. The above results show that any of the buffers tested was suitable in maintaining the number of WBCs at a very high level, proving the suitability of the reagent for the specific lysis of erythrocytes. Additionally, the pH values in the supernatant after the 1^{st} and 2^{nd} lysis were in the range of from 6.7 to 7.7, particularly of from 6.9 to 7.3.

### Comparative Example 7: Erythrocyte Lysis in different reagents with different lysis components

In a seventh test, the effectiveness of existing erythrocyte lysis protocols was tested as indicated. The results are shown in following table 7:

**Table 7: Effect of conventional erythrocyte lysis protocols on Recovery of White Blood Cells**

| | **Method** | **Testing conditions** | **Number of experiments** | **WBC recovery rate (Mean) [%]** | **Remarks** |
|---|---|---|---|---|---|
| 1 | Ammonium Chlorid based method | NH₄Cl (RCLB*); Dilution Blood: Lysis buffer = 1:2 | 10 | 63,2 | according to manufacturer's protocol |
| 2 | Ammonium Chlorid based method | NH₄Cl (RCLB*); Dilution Blood: Lysis buffer = 1:5 | 14 | 71,0 | according to manufacturer's protocol |
| 3 | Ammonium Chlorid based method | NH₄Cl (150 mM), 10 mM Tris pH 7,5; Dilution Blood: Lysis buffer = 1:5 | 1 | 35,9 | according to RCLB protocol |
| 4 | Ammonium Chlorid based method | NH₄Cl (150 mM), 5 mM Hepes; Dilution Blood: Lysis buffer = 1:6 | 1 | 35,5 | according to RCLB protocol |
| 5 | Ammonium Chlorid based method | NH₄Cl (75 mM)/ NaCl (0,45 %); Dilution Blood: Lysis buffer = 1:5 | 1 | 57,0 | according to RCLB protocol |
| 6 | Ammonium Chlorid based method | NH₄Cl (RCLB*); Dilution Blood: Lysis buffer = 1:1 | 1 | 30,0 | according to manufacturer's protocol |
| 7 | Ammonium Chlorid based method | NH₄Cl (RCLB*); Dilution Blood: Lysis buffer = 1:2; Washing of cells: 2 × RPMI | 2 | 55,5 | according to manufacturer's protocol |
| 8 | Acetic acid based lysis | Acetic acid (1%), Na₂CO₃, 4 ml blood; Dilution Blood: Acetic acid: Na₂CO₃ = 1:3:7,5 | 4 | 64,2 | protocol according to US 5,155,044 |
| 9 | Acetic acid based lysis | Acetic acid (1%), Na₂CO₃, 5 ml blood; Dilution Blood: Acetic acid: Na₂CO₃ = 1:3:7,5 | 2 | 70,0 | protocol according to US 5,155,044 (with modifications) |
| 10 | Acetic acid based lysis | Acetic acid (3%), Na₂CO₃; 5 ml blood; Dilution Blood: Acetic acid: Na₂CO₃ : 1:4:8 | 1 | 30,6 | protocol according to US 5,155,044 (with modifications) |
| 11 | Acetic acid based lysis | Acetic acid (3%), Na₂CO₃; 5 ml blood; Dilution Blood: Acetic acid: Na₂CO₃ : 1:3:6 | 1 | 52,8 | protocol according to US 5,155,044 (with modifications) |
| 12 | NaCl based lysis | NaCl (0,225%); 2 × lysis (1:5) | 2 | 52,5 | according to RCLB protocol |
| 13 | NaCl based lysis | NaCl (0,225%); 2 × lysis (1:9) | 1 | 60,0 | according to RCLB protocol |
| 14 | Saccharose/ Triton based lysis | Saccharose (320 mM); Tris (50 mM pH 7,5); MgCl₂ (5 mM); Triton (1 %) | 1 | complete lysis of all cells | protocol according to DE 102008032501 |
| 15 | Saccharose/ Triton based lysis | Saccharose (320 mM); Tris (12 mM pH 7,5); MgCl₂ (5 mM); Triton (1 %) | 1 | complete lysis of all cells | protocol according to DE 102008032501 |
| 16 | NaCl based lysis | Tris (10 mM pH 7,5); NaCl (12,45 mM); MgCl₂ (0,5 mM) | 1 | 24,2 | according to RCLB protocol |
| 17 | Piperidin-Hydrochlorid based lysis | Piperidin-Hydrochlorid (0,17 M); KHCO₃ (2,5 mM), Hepes (5mM) pH7,5; EDTA (0,1 mM); Washing of cells 2 × with PBS | 4 | 63,0 | protocol according to US 7,678,583 B2 |
| 18 | Piperidin-Hydrochlorid based lysis | Piperidin-Hydrochlorid (0,17 M); KHCO₃ (2,5 mM), Hepes (5mM) pH7,5; EDTA (0,1 mM); Washing of cells 2 × with Hepes | 1 | 0,0 | protocol according to US 7,678,583 B2 |
| 19 | Piperidin-Hydrochlorid based lysis | Piperidin-Hydrochlorid (0,128 M); KHCO₃ (2,5 mM), Hepes (5mM) pH7,5; EDTA (0,1 mM); Washing of cells 2 × with PBS | 1 | 66,0 | protocol according to US 7,678,583 B2 |
| 20 | Piperidin-Hydrochlorid based lysis | Piperidin-Hydrochlorid (0,085 M); KHCO₃ (2,5 mM), Hepes (5mM) pH7,5; EDTA (0,1 mM); Washing of cells 2 × with PBS | 1 | 43,3 | protocol according to US 7,678,583 B2 |
| 21 | Piperidin-Hydrochlorid based lysis | Piperidin-Hydrochlorid (0,17 M); KHCO₃ (2,5 mM), Hepes (5mM) pH7,5; EDTA (0,1 mM); Washing of cells 2 × with 0,9% NaCl | 1 | 64,1 | protocol according to US 7,678,583 B2 |
| 22 | NaCl based lysis | Tris (10 mM pH 7,5); NaCl (12,45 mM); MgCl₂ (0,5 mM) | 1 | 37,8 | according to RCLB protocol |
| 23 | Ammonium Chlorid based method | NH₄Cl (150 mM)/ Na-acetat (10mM); Dilution Blood: Lyis buffer = 1:5; pH 5 | 8 | 66,7 | according to RCLB protocol |
| 24 | Saponin based method | 0,02 % Saponin in PBS + 1 % BSA; Dilution Blood:Saponin = 1:1 | 2 | 40,1 | protocol according to US 5,840,515 (with modifications) |
| 24 | Saponin based method | 0,04 % Saponin in PBS + 1 % BSA; Dilution Blood:Saponin = 1:1 | 2 | 35,3 | protocol according to US 5,840,515 (with modifications) |

| | | | | | |
|---|---|---|---|---|---|
| * RCLB:Red Blood Cell Lysis buffer (Roche Applied Science, Cat. No. 11814389001): 150 mM NH4CI, 1 mM KHCO₃, 0.1 mM | | | | | |

The above results show that none of the buffers tested was suitable in maintaining the number of WBCs at a high level, proving the advantageous effect associated with the reagent as presented herein.

## Claims

1. Use of a reagent for the lysis of erythrocytes, the reagent being an aqueous solution comprising or consisting of HEPES ((4-(2-hydroxyethyl)-1-piperazineethanesulfonic acid), NH₄⁺/NH₃, a chelating agent, and optionally CO₃²⁻/HCO₃⁻wherein the final concentration during lysis of erythrocytes is in the range of
- from 2.5 mmol/l to 12 mmol/l HEPES,
- from 60 mmol/l to 120 mmol/l NH₄⁺/NH₃,
- from 0.04 mmol/l to 0.8 mmol/l chelating agent, and - from 0.15 mmol/l to 0.8 mmol/l CO₃²⁻/HCO₃⁻, if present,
wherein the chelating agent is selected from the group consisting of EGTA (ethylene glycol tetraacetic acid), BAPTA (1,2-bis(o-aminophenoxy)ethane-N,N,N',N'-tetraacetic acid), DTPA (diethylene triamine pentaacetic acid), EDTA (ethylenediamine tetraacetate) and NTA (N,N-bis(carboxymethyl)glycine).

2. The use of claim 1, wherein the reagent comprises from 0.15 mmol/l to 0.8 mmol/lCO₃²⁻/HCO₃⁻.

3. The use of claim 1 or 2, wherein the final concentration during lysis is in the range of
- from 3 mmol/l to 11 mmol/l HEPES,
- from 70 mmol/l to 100 mmol/l NH₄⁺/NH₃,
- from 0.05 mmol/l to 0.5 mmol/l chelating agent, and/or
- from 0.3 mmol/l to 0.6 mmol/l CO₃²⁻/HCO₃⁻, if present.

4. The use of any of claims 1 to 3, wherein the pH of the reagent is in the range of from 6.4 to 7.7.

5. The use of claim 4, wherein the pH is maintained in the range of from 6.4 to 7.7 during lysis of erythrocytes.

6. The use of any of claims 1 to 5, wherein the reagent is used in the detection, concentration or isolation of cells other than erythrocytes from a sample comprising erythrocytes.

7. The use of claim 6, wherein the sample is a blood sample.

8. The use of claim 6 or 7, wherein cells other than erythrocytes are leukocytes, circulating endothelial cells, or circulating tumor cells, particularly circulating tumor cells.

9. A method of lysing erythrocytes, the method comprising
a) providing a sample comprising erythrocytes; and
b) incubating the sample with the reagent for the lysis of erythrocytes, the reagent being an aqueous solution comprising or consisting of HEPES, NH₄⁺/NH₃, a chelating agent, and optionally CO₃²⁻/HCO3⁻,
wherein the final concentration during lysis of erythrocytes is in the range of
- from 2.5 mmol/l to 12 mmol/l HEPES,
- from 60 mmol/l to 120 mmol/l NH₄⁺/NH₃,
- from 0.04 mmol/l to 0.8 mmol/l chelating agent, and - from 0.15 mmol/l to 0.8 mmol/l CO₃²⁻/HCO₃⁻, if present,
wherein the chelating agent is selected from the group consisting of EGTA (ethylene glycol tetraacetic acid), BAPTA (1,2-bis(o-aminophenoxy)ethane-N,N,N',N'-tetraacetic acid), DTPA (diethylene triamine pentaacetic acid), EDTA (ethylenediamine tetraacetate) and NTA (N,N-bis(carboxymethyl)glycine).

10. The method of claim 9, wherein the reagent comprises from 0.15 mmol/l to 0.8 mmol/l CO₃²⁻/HCO₃⁻.

11. The method of claim 9 or 10 further comprising after step b) removing erythrocyte debris.

12. The method of any of claims 9 to 11, wherein the sample is a blood sample or a sample comprising erythrocytes and other cells.

13. The method of any of claims 9 to 12, wherein the method further comprises c) detecting or isolating cells other than erythrocytes from a sample comprising erythrocytes.

14. The method of claim 13, wherein cells other than erythrocytes are white blood cells or circulating tumor cells.

15. The method of any of claims 9 to 14, wherein the incubating of step b) is for at most 30 min.

## Patentansprüche

1. Verwendung eines Reagens zur Lyse von Erythrozyten, wobei das Reagens eine wässrige Lösung ist, die HEPES ((4-(2-Hydroxyethyl)-1-piperazinethansulfonsäure), NH₄⁺/NH₃, einen Chelatbildner und gegebenenfalls CO₃²⁻/HCO₃⁻ enthält oder daraus besteht, wobei die Endkonzentration während der Lyse der Erythrozyten im Bereich liegt von
- 2,5 mmol/l bis 12 mmol/l HEPES,
- 60 mmol/l bis 120 mmol/l NH₄⁺/NH₃,
- 0,04 mmol/l bis 0,8 mmol/l Chelatbildner, und
- 0,15 mmol/l bis 0,8 mmol/l CO₃²⁻/HCO₃⁻, falls vorhanden,
wobei der Chelatbildner ausgewählt ist aus der Gruppe bestehend aus EGTA (Ethylenglykoltetraessigsäure), BAPTA (1,2-bis(o-Aminophenoxy)ethan-N,N,N',N'-tetraessigsäure), DTPA (Diethylentriaminpentaessigsäure), EDTA (Ethylendiamintetraacetat) und NTA (N,N-Bis(carboxy¬methyl)glycin).

2. Verwendung nach Anspruch 1, wobei das Reagenz 0,15 mmol/l bis 0,8 mmol/l CP₃²⁻/HCO₃⁻ enthält.

3. Verwendung nach Anspruch 1 oder 2, wobei die Endkonzentration während der Lyse im Bereich liegt von
- 3 mmol/l bis 11 mmol/l HEPES,
- 70 mmol/l bis 100 mmol/l NH₄⁺/NH₃,
- 0,05 mmol/l bis 0,5 mmol/l Chelatbildner und/oder
- 0,3 mmol/l bis 0,6 mmol/l CO₃²⁻/HCO₃⁻, falls vorhanden.

4. Verwendung nach einem der Ansprüche 1 bis 3, wobei der pH-Wert des Reagens im Bereich von 6,4 bis 7,7 liegt.

5. Verwendung nach Anspruch 4, wobei der pH-Wert während der Lyse der Erythrozyten in einem Bereich von 6,4 bis 7,7 gehalten wird.

6. Verwendung nach einem der Ansprüche 1 bis 5, wobei das Reagenz für den Nachweis, die Konzentrierung oder die Isolierung von anderen Zellen als Erythrozyten aus einer Erythrozyten enthaltenden Probe verwendet wird.

7. Verwendung nach Anspruch 6, wobei die Probe eine Blutprobe ist.

8. Verwendung nach Anspruch 6 oder 7, wobei andere Zellen als Erythrozyten Leukozyten, zirkulierende Endothelzellen oder zirkulierende Tumorzellen, insbesondere zirkulierende Tumorzellen, sind.

9. Verfahren zum Lysieren von Erythrozyten, wobei das Verfahren umfasst
a) Bereitstellen einer Probe, die Erythrozyten enthält, und
b) Inkubieren der Probe mit dem Reagenz für die Lyse von Erythrozyten, wobei das Reagens eine wässrige Lösung ist, die HEPES, NH₄⁺/NH₃, einen Chelatbildner und gegebenenfalls CO₃²⁻/HCO₃⁻ enthält oder daraus besteht, wobei die Endkonzentration während der Lyse der Erythrozyten im Bereich liegt von
- 2,5 mmol/l bis 12 mmol/l HEPES,
- 60 mmol/l bis 120 mmol/l NH₄⁺/NH₃,
- 0,04 mmol/l bis 0,8 mmol/l Chelatbildner, und
- 0,15 mmol/l bis 0,8 mmol/l CO₃²⁻/HCO₃⁻, falls vorhanden,
wobei der Chelatbildner ausgewählt ist aus der Gruppe bestehend aus EGTA (Ethylenglykoltetraessigsäure), BAPTA (1,2-Bis(o-aminophenoxy)ethan-N,N,N',N'-tetraessigsäure), DTPA (Diethylentriaminpentaessigsäure), EDTA (Ethylendiamintetraacetat) und NTA (N,N-Bis(carboxy-imethyl)glycin).

10. Verfahren nach Anspruch 9, wobei das Reagenz 0,15 mmol/l bis 0,8 mmol/l CO₃²⁻/HCO₃⁻ enthält.

11. Verfahren nach Anspruch 9 oder 10, weiterhin nach Schritt b) umfassend das Entfernen von Erythrozytendebris.

12. Verfahren nach einem der Ansprüche 9 bis 11, wobei die Probe eine Blutprobe oder eine Probe ist, die Erythrozyten und andere Zellen enthält.

13. Verfahren nach einem der Ansprüche 9 bis 12, wobei das Verfahren ferner umfasst c) Nachweisen oder Isolieren von anderen Zellen als Erythrozyten aus einer Erythrozyten umfassenden Probe.

14. Verfahren nach Anspruch 13, wobei andere Zellen als Erythrozyten weiße Blutzellen oder zirkulierende Tumorzellen sind.

15. Verfahren nach einem der Ansprüche 9 bis 14, wobei das Inkubieren von Schritt b) höchstens 30 Minuten dauert.

## Revendications

1. Utilisation d'un réactif de lyse des érythrocytes, le réactif étant une solution aqueuse comprenant ou constituée par de l'HEPES (acide (4-(2-hydroxyéthyl)-1-pipérazineéthanesulfonique), NH₄⁺/NH₃, un agent chélatant, et éventuellement CO₃²⁻/HCO₃⁻
où la concentration finale pendant la lyse des érythrocytes se situe dans l'intervalle
- de 2,5 mmol/l à 12 mmol/l de HEPES,
- de 60 mmol/l à 120 mmol/l de NH₄⁺/NH₃,
- de 0,04 mmol/l à 0,8 mmol/l d'agent chélatant, et
- de 0,15 mmol/l à 0,8 mmol/l de CO₃²⁻/HCO₃⁻, si présent,
où l'agent chélatant est sélectionné dans le groupe consistant en EGTA (acide éthylèneglycoltétraacétique), BAPTA (acide 1,2-bis(o-aminophénoxy)éthane-N,N,N',N'-tétraacétique), DTPA (acide diéthylènetriaminepentaacétique), EDTA (éthylènediaminetétraacétate) et NTA (N,N-bis(carboxyméthyl)glycine).

2. Utilisation selon la revendication 1, où le réactif comprend de 0,15 mmol/l à 0,8 mmol/l de CO₃²⁻/HCO₃⁻.

3. Utilisation selon la revendication 1 ou 2, où la concentration finale pendant la lyse se situe dans l'intervalle
- de 3 mmol/l à 11 mmol/l de HEPES,
- de 70 mmol/l à 100 mmol/l de NH₄⁺/NH₃,
- de 0,05 mmol/l à 0,5 mmol/l d'agent chélatant, et/ou
- de 0,3 mmol/l à 0,6 mmol/l de CO₃²⁻/HCO₃⁻, si présent.

4. Utilisation selon l'une quelconque des revendications 1 à 3, où le pH du réactif se situe dans l'intervalle de 6,4 à 7,7.

5. Utilisation selon la revendication 4, où le pH est maintenu dans l'intervalle de 6,4 à 7,7 pendant la lyse des érythrocytes.

6. Utilisation selon l'une quelconque des revendications 1 à 5, où le réactif est utilisé pour la détection, la concentration ou l'isolement de cellules différentes des érythrocytes à partir d'un échantillon comprenant des érythrocytes.

7. Utilisation selon la revendication 6, où l'échantillon est un échantillon de sang.

8. Utilisation selon la revendication 6 ou 7, où les cellules différentes des érythrocytes sont des leucocytes, des cellules endothéliales circulantes, ou des cellules tumorales circulantes, en particulier des cellules tumorales circulantes.

9. Procédé de lyse des érythrocytes, le procédé comprenant
a) se procurer un échantillon comprenant des érythrocytes ; et
b) incuber l'échantillon avec le réactif de lyse des érythrocytes, le réactif étant une solution aqueuse comprenant ou constituée par de l'HEPES, NH₄⁺/NH₃, un agent chélatant, et éventuellement CO₃²⁻/HCO₃⁻,
où la concentration finale pendant la lyse des érythrocytes se situe dans l'intervalle
- de 2,5 mmol/l à 12 mmol/l de HEPES,
- de 60 mmol/l à 120 mmol/l de NH₄⁺/NH₃,
- de 0,04 mmol/l à 0,8 mmol/l d'agent chélatant, et
- de 0,15 mmol/l à 0,8 mmol/l de CO₃²⁻/HCO₃⁻, si présent,
où l'agent chélatant est sélectionné dans le groupe consistant en EGTA (acide éthylèneglycoltétraacétique), BAPTA (acide 1,2-bis(o-aminophénoxy)éthane-N,N,N',N'-tétraacétique), DTPA (acide diéthylènetriaminepentaacétique), EDTA (éthylènediaminetétraacétate) et NTA (N,N-bis(carboxyméthyl)glycine).

10. Procédé selon la revendication 9, dans lequel le réactif comprend de 0,15 mmol/l à 0,8 mmol/l de CO₃²⁻/HCO₃⁻.

11. Procédé selon la revendication 9 ou 10 comprenant en outre après l'étape b) à éliminer les débris d'érythrocytes.

12. Procédé selon l'une quelconque des revendications 9 à 11, dans lequel l'échantillon est un échantillon de sang ou un échantillon comprenant des érythrocytes et d'autres cellules.

13. Procédé selon l'une quelconque des revendications 9 à 12, lequel procédé comprenant en outre à
c) détecter ou isoler des cellules différentes des érythrocytes à partir d'un échantillon comprenant des érythrocytes.

14. Procédé selon la revendication 13, dans lequel les cellules différentes des érythrocytes sont des globules blancs ou des cellules tumorales circulantes.

15. Procédé selon l'une quelconque des revendications 9 à 14, dans lequel l'incubation de l'étape b) se fait pendant au plus 30 min.
